# EUROPÄISCHE PATENTSCHRIFT

(11) **EP 1 105 263 B1**
(45) Veröffentlichungstag und Bekanntmachung des Hinweises auf die Patenterteilung: **20.11.2002**
(21) Anmeldenummer: 99940110.2
(22) Anmeldetag: 02.08.1999
(51) Int. Cl.: B26D 7/18, B26F 1/38, A61F 13/00, A61K 9/00, A61L 15/00

(54) **VERFAHREN ZUM HERSTELLEN VON KLEBENDEN STANZLINGEN AUS EINER ENDLOSEN BAHN UND NACH DEM VERFAHREN HERGESTELLTER STANZLING**
METHOD FOR PRODUCING ADHESIVE BLANKS FROM AN ENDLESS BAND AND BLANKS OBTAINED ACCORDING TO SAID METHOD
PROCEDE DE FABRICATION DE DECOUPES ADHESIVES A PARTIR D'UNE BANDE CONTINUE ET DECOUPES OBTENUES SELON LEDIT PROCEDE

(30) Priorität: 20.08.1998 DE 19837764
(43) Veröffentlichungstag der Anmeldung: 13.06.2001
(73) Patentinhaber: LTS Lohmann Therapie-Systeme AG, 56626 Andernach (DE)
(72) Erfinder: HARDT, Frank, D-53547 Hausen (DE); GENICH, Paul, D-56626 Andernach (DE)
(74) Vertreter: Flaccus, Rolf-Dieter, Dr.
(86) Internationale Anmeldenummer: EP9905562
(87) Internationale Veröffentlichungsnummer: WO00010781

(56) Entgegenhaltungen:
- WO-A-97/14126
- GB-A- 532 018
- GB-A- 865 165
- GB-A- 2 019 807
- GB-A- 2 218 682
- US-A- 4 746 394
- US-A- 4 853 063
- US-A- 5 244 677

## Beschreibung

Die Erfindung betrifft ein Verfahren zum Herstellen von klebenden Stanzlingen aus einer endlosen Bahn mit mindestens einer, gegebenenfalls ein Füllgut enthaltenden, inneren Aussparung, wobei die Bahn eine Trägerschicht mit mindestens einer Klebschicht aufweist. Bei den klebenden Stanzlingen kann es sich um ein- oder zweiseitig klebende Dichtungsringe oder um mit Löchern versehene Etiketten handeln. Es kann sich auch um Stanzlinge nach der Art der unter dem Namen "Power-Strips" der Firma Beiersdorf erhältlichen Produkte handeln, die zusätzlich mit einer Aussparung versehen sind.

In solche, aus bahnförmigem Material herzustellende Gegenstände werden nach dem Stand der Technik mittels einer sogenannten Lochpfeife (auch als Vollwerkzeug bezeichnet) entsprechend geformte Aussparungen eingebracht. Das dabei ausgestanzte Material wird in der Pfeife nach oben gedrückt. Nach mehrmaligem. Ausstanzen der Aussparung ist es erforderlich, die materialgefüllte Pfeife mechanisch von dem ausgestanzten Material zu befreien. Hiermit verbunden sind Unterbrechungen einer kontinuierlichen Prozeßführung. Ein weiterer Nachteil ist die Tatsache, daß ein solches Vollwerkzeug eine mechanisch anspruchsvolle, daher recht störanfällige und auch teure Vorrichtung ist.

In GB 2,218,682 wird ein Verfahren zur Herstellung von klebenden Stanzlingen mit inneren Aussparungen aus einer endlosen Bahn beschrieben, wobei die Bahn eine Trägerschicht und mindestens eine Klebeschicht aufweist, das Stanzen der Kontur der inneren Aussparung aus der Klebeschicht ohne Durchstanzen der Trägerschicht erfolgt und beim Stanzen der äußeren Kontur diese keinen gemeinsamen Punkt mit der Kontur der inneren Aussparung besitzt. Dieser Verfahren bildet die Grundlage für den Oberbegriff des Anspruchs 1.

Bei den klebenden Stanzlingen kann es sich bevorzugt um lokal, regional oder systemisch wirkende Pflaster mit einer Rückschicht, einer Klebschicht und einem Wirkstoffreservoir handeln, das bis zur Applikation mit einer ablösbaren Schutzschicht versehen ist.

Es sind auf die Haut aufzubringende Arzneiformen mit dem Aussehen traditioneller Pflaster bekannt, welche an die Haut abzugebende Arzneistoffe enthalten und z.B. als Hühneraugenpflaster bekannt sind. Ein solches System kann einen oder mehrere Arzneistoffe enthalten.

Beispielsweise offenbart die Patentschrift US-A 5,244,677 eine dermale Applikationsform aus einem schaumartigen Material oder einem nicht verwobenen Vlies-Streifen, die eine innere Aussparung aufweist. handeln, die bis zur Applikation mit einer ablösbaren Schutzschicht versehen sind.

Es sind auf die Haut aufzubringende Arzneiformen mit dem Aussehen traditioneller Pflaster bekannt, welche an die Haut abzugebende Arzneistoffe enthalten und z. B. als Hühneraugenpflaster bekannt sind. Ein solches System kann einen oder mehrere Arzneistoffe enthalten.

Der Erfindung liegt somit die Aufgabe zugrunde, ein Verfahren zur Verfügung zu stellen, das die Herstellung von klebenden Stanzlingen mit einer inneren Aussparung (= ein "Loch") aus einer endlosen Bahn unter Vermeidung der Verwendung einer Lochpfeife ermöglicht.

Außerdem liegt der Erfindung die Aufgabe zugrunde, klebende Stanzlinge mit einer inneren Aussparung zur Verfügung zu stellen. Eine weitere Aufgabe der Erfindung besteht darin, klebende Stanzlinge in der Form von wirkstoffhaitigen Pflastern zur Verfügung zu stellen, die eine mit Füllgut befüllte innere Aussparung besitzen.

Gelöst wird die Aufgabe durch ein Verfahren gemäß Anspruch 1.

Dabei kann anstelle der Abziehkante (12) auch eine Abziehrolle verwendet werden, die ein technisches Äquivalent darstellt. Zur Klarstellung: Die ausgesparten Stanzreste (7) stellen das Material dar, das nach seiner Entfernung aus der endlosen Bahn (1) die inneren Aussparungen (3) (= die Löcher) darin hinterlassen.

Weitere Schritte zur Herstellung der mit Füllgut befüllten, wirkstoffhaltigen Pflaster sind:
e) Füllen der inneren Aussparung (3) mit Füllgut (2) und Aufkaschieren einer Abdeckfolie (9),
f) Konturstanzen der äußeren Pflasterform unter Vereinzelung der Stanzlinge und Abgittern der Stanzreste.

Das erfindungsgemäße Herstellungsverfahren ist überraschend unkompliziert, ohne sogenannte Lochpfeife durchführbar und infolgedessen ausgesprochen kostengünstig.

Eine Ausgestaltung des Verfahrens sieht vor, daß die Schritte in der Reihenfolge a) bis d), gegebenenfalls bis f) durchgeführt werden. Sie können aber auch, abweichend hiervon, in der Reihenfolge b), a), c), d), gegebenenfalls zusätzlich e) und f) durchgeführt werden.

Eine weitere Vereinfachung besteht darin, daß die Schritte a) und b) gleichzeitig miteinander durchgeführt werden, z. B. mit einer Hubstanze oder einer Schneidewalze. Diese Stanzwerkzeuge besitzen dann eine Oberflächenstruktur mit Schnittkanten, die die äußere Kontur der Stanzlinge und die Kontur der inneren Aussparung aufweisen.

Schließlich können die Arbeitsschritte in der Reihenfolge a), c), d), b), gegebenenfalls zusätzlich e) und f) durchgeführt werden.

Die Schritte a) bis d) können simultan erfolgen, so daß das Verfahren kontinuierlich mit konstanter oder variabler Geschwindigkeit abläuft. Das Verfahren kann aber auch diskontinuierlich verlaufen, wenn nämlich die Verwendung einer Hubstanze das Anhalten der endlosen Bahn (1) erforderlich macht. Bevorzugt ist ein solches diskontinuierliches Verfahren, wenn der zusätzliche Schritt e) relativ zeitaufwendig ist.

Eine weitere vorteilhafte Verfahrensvariante ist dadurch gekennzeichnet, daß die innere Aussparung (3) der Klebschicht (5) durch die Matrixschicht (6) hindurch und zusammen mit dieser ausgestanzt wird.

Die geometrische Form der inneren Aussparung (3) spielt keine Rolle. Bevorzugt ist eine kreisrunde Form. Wichtig ist, daß die Dicke der Klebschicht (5) und gegebenenfalls der Matrixschicht (6) zusammen kleiner als die maximale Ausdehnung der inneren Aussparung (3) in Längs- und / oder Querrichtung ist.

Ein nach dem Verfahren hergestellter klebender Stanzling hat die folgenden Merkmale :
a) eine Klebschicht (5) mit einer inneren Aussparung (3) und
b) eine die Klebeschicht (5) und die innere Aussparung (3) abdeckende, wiederablösbare Schutzfolie (8).

Zusätzlich kann dieser klebende Stanzling eine Matrixschicht (6) besitzen. Diese Matrixschicht kann eine zur der inneren Aussparung (3) in der Klebschicht (5) kongruente (flächenförmig identische) innere Aussparung besitzen.

Der klebende Stanzling kann eine auf der Klebschicht (5) oder auf der sich darüber befindenden Matrixschicht (6) haftende Abdeckschicht (9) besitzen. Die innere Aussparung (3) kann gegebenenfalls mit einem Füllgut (2) befüllt sein, das vorzugsweise eine wirkstoffhaltige Masse ist.

Als Füllgut (2) kommen fließ- oder schüttfähige Zubereitungen in Frage, also flüssige, halbfeste, gelartige, pastöse, pulverförmige oder schmelzbare Stoffe oder Mischungen. Als Stoffe kommen z. B. topisch (lokal und / oder regional) und / oder systemisch wirkende pharmazeutische Wirkstoffe in Frage, unter anderen Salicylsäure, Milchsäure, 5-Fluoruracil, Capsaicin, Acetylsalicylsäure, Nonylsäurevanillylamid etc.

Geeignete Materialien für die Klebschicht (5) sind natürliche oder künstliche Polymere mit haftklebenden Eigenschaften, die dem Fachmann bekannt sind, z. B. Polyacrylate, Polyisobutylene, Silicone, Kautschuk, SIS-Block-Copolymere etc. Gegebenenfalls muß die Klebrigkeit durch Zugabe von Klebharzen ("tackyfier") den Erfordernissen angepaßt werden.

Für die Matrixschicht (6) kommen ebenfalls bevorzugt natürliche und künstliche Polymere wie Polyacrylate, Polyethylene, Polypropylene, Polybutylene, Polyurethane, Poly-1-buten, Polyisobuten, Kautschuke, Silikonkautschuke, Cellulose, Zellstoff, Papier, Baumwolle, Acetylcellulose, Celluloid, Viscose, Polyacrylnitril, Polyvinylalkohol, Polyvinylacetat, Polyvinylether, Ethylen-Vinylacetat-Copolymer (EVA) und dergleichen in Frage. Diese Materialien und folglich die Matrixschicht (6) sind nicht klebend. Sie können als kompaktes Material, Schaum, Gewebe, poröse Folie, Vlies etc. vorliegen.

Das Verfahren und die zu dessen Durchführung vorgesehene Vorrichtung sind unkompliziert, übersichtlich und funktionell überraschend wirksam. Insoweit löst die Erfindung in optimaler Weise die eingangs gestellte Aufgabe.

Weitere Einzelheiten, Merkmale und Vorteile der Erfindung ergeben sich aus den nachfolgenden Ausführungsbeispielen und den dargestellten Zeichnungen.

### BEISPIEL 1: Herstellung eines befüllten Stanzlings

Bei der kontinuierlichen Herstellung der klebenden Stanzlinge (11) aus einer endlosen Bahn (1) wird diese in Richtung des Pfeiles 13 zunächst in eine Stanzstation (15) gebracht, worin die Konturen (17) der inneren Aussparungen (3) aus mindestens der Klebschicht (5) durchstanzt werden, ohne daß jedoch die Trägerschicht (4) mit durchstanzt wird. Im gezeigten Beispiel befindet sich auf der Klebschicht (5) noch eine weitere, optionale Schicht, nämlich die Matrixschicht (6). Im vorliegenden Beispiel wird sowohl die Matrixschicht (6) als auch die Klebschicht (5) in der Stanzstation (15) durchstanzt, wobei jedoch der ausgesparte Stanzrest (7) (= der "Stanzkern") zunächst noch innerhalb der Kontur (17) der inneren Aussparung (3) verbleibt. Geeignete Stanzstationen (15) sind entweder eine Hubstanze, die eine Verfahrensweise mit einem Stop der Bahn ermöglicht, oder eine rotierende Schneidwalze, die eine variable oder konstante Bahngeschwindigkeit ohne Stop der Bahn ermöglicht. Ein energiereicher Laserstrahl, der die Kontur (17) der inneren Aussparung (3) abfährt, kann ebenfalls die technische Funktion der Stanzstation (15) ausüben.

Beim weiteren Transport in Richtung des Pfeiles 13 wird dann in der zweiten Schneidstation (18) die äußere Kontur (10) der Stanzlinge (11) aus der Matrixschicht (6) und der Klebschicht (5) ausgestanzt, wobei deren äußere Kontur (10) keinen gemeinsamen Punkt mit der Kontur (17) der inneren Aussparung (3) aufweist. Ein dabei gegebenenfalls entstandenes Stanzgitter wird "abgegittert". Sodann erreicht die so vorgestanzte endlose Bahn (1) die Abziehkante (12), an der die nicht durchgestanzte Trägerschicht (4) zusammen mit den ausgesparten Stanzresten (7) abgezogen wird.

Die so vorbereiteten Stanzlinge werden unmittelbar danach über eine Rolle (19) auf eine Schutzfolie (8) übertragen, wobei die Unterseite der Klebschicht (5) mit der Schutzfolie (8) abgedeckt wird.

Beim weiteren Transport der konturgestanzten Stanzlinge mit noch offenen (d. h. nicht abgedeckten und nicht befüllten) inneren Aussparungen (3) erreichen diese eine Position unterhalb der Füllstation (16), welche jeweils eine dosierbare Portion an Füllgut (2) in die bis dahin offenen inneren Aussparungen (3) einfüllt. Danach wird auf die Stanzlinge beim weiteren Transport von oben eine Abdeckfolie (9) in einer Kaschierstation (20) aufkaschiert.

### BEISPIEL 2: Herstellung eines befüllten Stanzlings

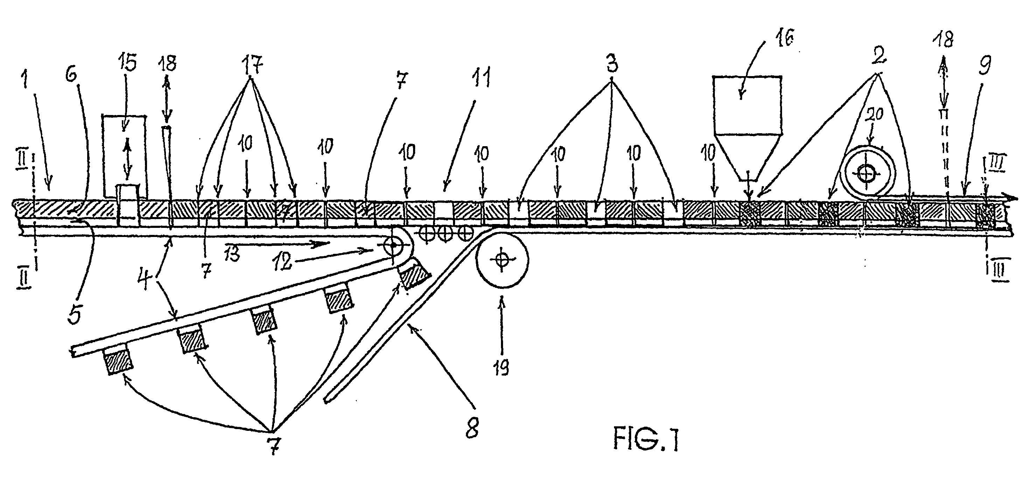

Wie Beispiel 1, jedoch erfolgt die Stanzung der äußeren Kontur (10) des klebenden Stanzlings (11) nach dem Einfüllen des Füllguts (2), das heißt die zweite Schneidstation (18) (in FIG.1 gestrichelt dargestellt) befindet sich hinter Füllstation (16) und Kaschierstation (20).

### BEISPIEL 3: Herstellung eines nicht-befüllten Stanzlings

Wie Beispiel 1, jedoch erfolgen Stanzung der Kontur (17) der inneren Aussparung (3) und der äußeren Kontur (10) des klebenden Stanzlings gleichzeitig in einer rotativen Schneidwalze. Aufgrund der geometrischen Form der äußeren Kontur des Stanzlings fällt dabei kein Stanzgitter an. Ein Abgittern ist daher nicht erforderlich. Beim anschließenden Übertragen der vorgestanzten Stanzlinge auf die Schutzfolie (8) erfolgt eine Vereinzelung, da diese Schutzfolie eine höhere Bahngeschwindigkeit besitzt als die endlose Bahn (1). Ein Befüllen der inneren Aussparung (3) erfolgt nicht.

### BEISPIEL 4: Herstellung eines wirkstoffhaltigen Pflasters

Die Herstellung eines wirkstoffhaltigen Pflasters erfolgt auf einer Flachbettstanze, welche mit folgenden Aggregaten ausgerüstet ist:
- zweite Ebene mit Stanzstation
- Kaschiervorrichtung
- Dosierstation
- Formatstanze
- Querschneider
- Verpackungseinheit mit Heißsiegelstanze (bei on line-Verpackung)

Die Schutzfolie wird in die Maschine eingezogen. Auf einer zweiten Ebene führt man das Polyethylenschaum-Laminat einer Stanze zu, welche die Löcher für die Befüllung mit Wirkstoffmasse ausstanzt. Das Zwischenabdeckpapier mit dem daran haftenden, ausgestanzten Schaum wird abgezogen.

Der gelochte Schaum wird auf die Schutzfolie aufkaschiert und in der Dosierstation mit Wirkstoffmasse befüllt. Nach Entfernen des zwischenabdeckpapiers vom Laminat wird die kleberbeschichtete, hautfarbene Polyethylenfolie auf den Schaum aufkaschiert.

In dieses Laminat, bestehend aus Schutzfolie, Kleber, Schaum mit Wirkstoffmasse, Kleber und hautfarbener Polyethylenfolie wird mittels Flachbettstanze mit einem Bandstahlschnitt die Pflasterkontur von oben eingestanzt. Überschüssige Polyethylenfolie und Schaum werden nach oben abgezogen, wobei die einzelnen Pflaster auf der Schutzfolie kleben bleiben. Nach dem Querschneiden werden die Pflaster zweinutzig in Packstoff eingesiegelt.

### BEISPIEL 5: Herstellung klebender Stanzlinge mit rechteckiger innerer Aussparung

Eine aus einem Laminat (enthaltend eine 0,9 mm dicke Polyethylenmatrix, eine 50 •m dicke Polyacrylatklebschicht und eine silikonisierte Papierträgerschicht) bestehende, endlose Bahn von 3 cm Breite wird in eine rotative Schneidwalze geführt, die rechteckige innere Aussparungen (3) von 1,6 cm Länge und 6 mm Breite ausstanzt. Gleichzeitig wird durch diese Schneidwalze ein Schnitt in 6 cm Abstand in die endlose Bahn gestanzt, und zwar so, daß in den vorgestanzten Stanzlingen die innere Aussparung (3) in der Mitte liegt. Ein Abgittern ist nicht erforderlich, da kein überstehender Rand zwischen den Stanzlingen bzw. an deren seitlichen Begrenzungen befindlich ist. Die vorgestanzten Stanzlinge werden an einer Umlenkrolle von der Trägerschicht (4) und dem ausgesparten Stanzrest (7) befreit. Beim Übertragen auf die mit höherer Geschwindigkeit laufende silikonisierte Polyethylenterephthalat-Schutzfolie (8) entstehen Abstände von 1 cm zwischen den vereinzelten Stanzlingen.

### Beschreibung der Abbildungen:

FIG.1 zeigt das Schema einer Vorrichtung zur Durchführung des Verfahrens zum Herstellen von klebenden Stanzlingen aus einer endlosen Bahn.
FIG. 2a zeigt im Schnitt der Ebene II-II die aus der Trägerschicht (4), der Klebschicht (5) und der Matrixschicht (6) zusammengesetzte endlose Bahn (1).

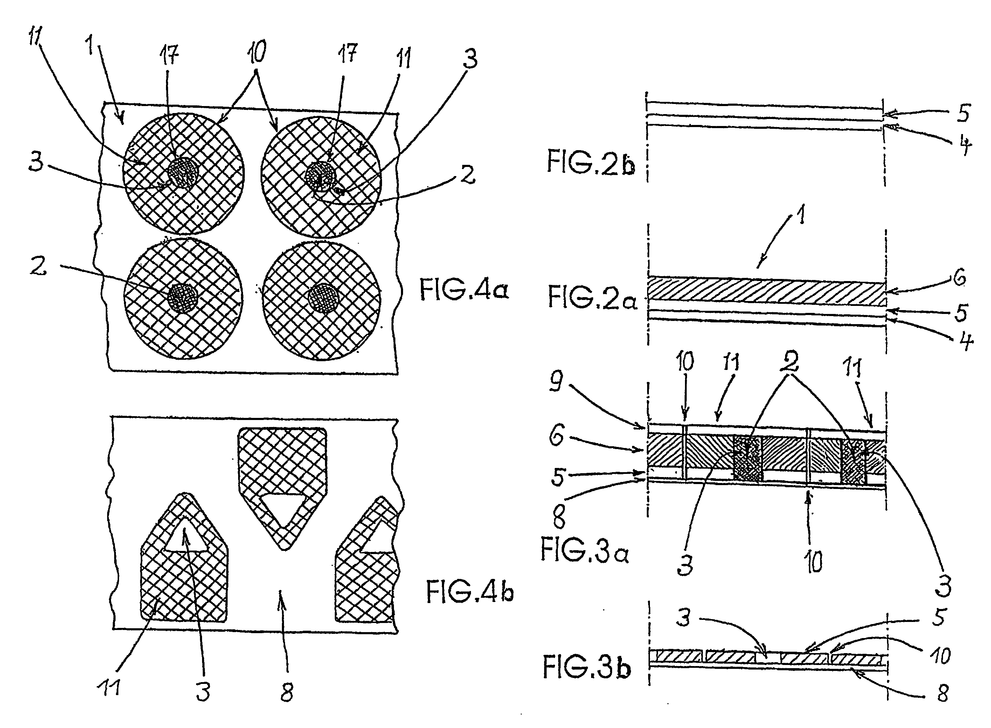

FIG. 2b zeigt eine aus Trägerfolie (4) und Klebschicht (5) bestehende Bahn (1)
FIG. 3a zeigt im Schnitt der Ebene III-III die Struktur der klebenden Stanzlinge (11) nach Stanzen der Konturen (17) der inneren Aussparungen (3) und der äußeren Konturen (10) sowie nach Befüllen der inneren Aussparungen (3) mit Füllgut (2) und Aufkaschieren einer Abdeckfolie (9).
FIG. 3b zeigt einen aus einer Klebschicht (5) bestehenden, die innere Aussparung (3) enthaltenden Stanzling auf der zweiten Trägerfolie (8) ohne Füllmaterial.
FIG. 4a zeigt in Draufsicht innerhalb der äußeren Konturen 10 Stanzlinge 11 mit Stanzkonturen 17 der inneren Aussparungen (3) sowie das in den inneren Aussparungen (3) enthaltene Füllgut (2). Die Stanzlinge 11 befinden sich vor der Vereinzelung noch innerhalb der endlosen Bahn (1).
FIG. 4b zeigt klebende Stanzlinge 11 in Draufsicht, bei denen die innere Aussparung (3) nicht befüllt ist.

## Patentansprüche

1. Verfahren zum Herstellen von klebenden Stanzlingen (11) mit inneren Aussparungen (3) aus einer endlosen Bahn (1) wobei die Bahn eine Trägerschicht (4) und mindestens eine Klebschicht (5) aufweist, mit den folgenden Schritten:
a) Stanzen der Kontur (17) der inneren Aussparung aus der Klebschicht, wobei jedoch die Trägerschicht nicht durchstanzt wird,
b) Stanzen der äußeren Kontur (10) der Stanzlinge aus der Klebschicht, wobei diese äußere Kontur keinen gemeinsamen Punkt mit der Kontur der inneren Aussparung besitzt, **gekennzeichnet durch** die Schritte:
c) Umlenken der Trägerschicht (4) über eine Abziehkante (12) oder Abziehrolle, wobei ausgesparte Stanzreste (7) der inneren Aussparung zusammen mit der Trägerschicht abgezogen werden,
d) Übertragung der von der Trägerschicht und den ausgesparten Stanzresten (7) befreiten Bahn auf eine Schutzfolie (8).

2. Verfahren nach Anspruch 1, **gekennzeichnet durch** den weiteren Schritt:
e) Füllen der inneren Aussparung (3) mit Füllgut (2) und Aufkaschieren einer Abdeckfolie.

3. Verfahren nach Anspruch 1 oder 2, **gekennzeichnet durch** den weiteren Schritt:
f) Konturstanzen der äußeren Pflasterform unter Vereinzelung der Stanzlinge und Abgittern der Stanzreste.

4. Verfahren nach Anspruch 1, **dadurch gekennzeichnet, daß** die Schritte in der Reihenfolge a) bis d) durchgeführt werden.

5. Verfahren nach Anspruch 1, **dadurch gekennzeichnet, daß** die Schritte in der Reihenfolge b), a), c) und d) durchgeführt werden.

6. Verfahren nach Anspruch 1, **dadurch gekennzeichnet, daß** die Schritte a) und b) gleichzeitig durchgeführt werden.

7. Verfahren nach Anspruch 1, **dadurch gekennzeichnet, daß** die Schritte in der Reihenfolge a), c), d) und b) durchgeführt werden.

8. Verfahren nach Anspruch 1, wobei die endlose Bahn (1) eine Trägerschicht (4), eine Klebschicht (5) und eine Matrixschicht (6) enthält, **dadurch gekennzeichnet, daß** die innere Aussparung (3) der Klebschicht (5) durch die Matrixschicht (6) hindurch und zusammen mit dieser ausgestanzt wird.

9. Verfahren nach einem der Ansprüche 1 bis 8, **dadurch gekennzeichnet, daß** die innere Aussparung (3) mit Füllgut (2) befüllt wird, die eine fließ- oder schüttfähige Zubereitung mit einem pharmazeutischen Wirkstoff enthält, der zu einer lokalen, regionalen, topischen und/oder systemischen Wirkung befähigt ist.

10. Verfahren nach Anspruch 9, **dadurch gekennzeichnet, daß** der pharmazeutische Wirkstoff aus der Gruppe umfassend Salicylsäure, Milchsäure, 5-Fluoruracil, Capsaicin, Acetylsalicylsäure oder Nonylsäurevanillylamid ausgewählt ist.

## Claims

1. Method for producing adhesive blanks (11) having an inner recess (3), from an endless band (1), said band comprising a carrier layer (4) and at least one adhesive layer (5), said method comprising the steps:
a) punching of the contour (17) of the inner recess out of the adhesive layer, without, however, punching through the carrier,
b) punching of the outer contour (10) of the blanks out of the adhesive layer, said outer contour not having any point in common with the contour of the inner recess,
and being **characterized by** the steps:
c) deflecting the carrier layer (4) around a stripping edge (12) or a stripping role, with the recessed residual punched portions (7) of the inner recesses being stripped off along with the carrier layer,
d) transferring the band, which has been freed of the carrier layer and of the recessed residual punched portions (7), onto a protective film (8).

2. Method according to Claim 1, **characterized by** the further step of:
e) filling the inner recess (3) with feed material (2) and laminating a cover film thereon.

3. Method according to Claim 1 or 2, **characterized by** the further step of:
f) contour punching of the outer shape of the plaster while separating the blanks and separating the residual punched portions by lattice.

4. Method according to Claim 1, **characterized in that** the steps are performed in the order of a) to d).

5. Method according to Claim 1, **characterized in that** the steps are performed in the order of b), a), c) and d).

6. Method according to Claim 1, **characterized in that** steps a) and b) are performed simultaneously.

7. Method according to Claim 1, **characterized in that** the steps are performed in the order of a), c), d) and b).

8. Method according to Claim 1, where the endless band (1) comprises a carrier layer (4), an adhesive layer (5) and a matrix layer (6), **characterized in that** the inner recess (3) of the adhesive layer (5) is through-punched through the matrix layer (6) and is punched out together with said matrix layer.

9. Method according to any one of Claims 1 to 8, **characterized in that** the inner recess (3) is filled with a feed material (2) containing a flowable or pourable preparation which comprises a pharmaceutically active substance capable of local, regional, topic and/or systemic action.

10. Method according to Claim 9, **characterized in that** the pharmaceutically active substance is selected from the group comprising salicylic acid, lactic acid, 5-fluorouracil, capsaicin, acetylsalicylic acid, or nonylic acid vanillyl amide.

## Revendications

1. Procédé de fabrication de découpes adhésives (11) avec évidements intérieurs (3) à partir d'une bande continue (1), ladite bande ayant une couche support (4) et au moins une couche adhésive (5), comprenant les étapes suivantes :
a) découpage du contour (17) de l'évidement intérieur dans la couche adhésive, sans toutefois passer à travers la couche support,
b) découpage du contour extérieur (10) des découpes dans la couche adhésive, ce contour extérieur ne possédant PAS DE point commun avec le contour de l'évidement intérieur,
**caractérisé par** les étapes :
c) retournement de la couche support (4) sur un bord détacheur (12) ou rouleau détacheur, les résidus de découpage (7) de l'évidement intérieur étant détachés ensemble avec la couche support,
d) transfert de la bande débarrassée de la couche support et des résidus de découpage (7) sur une feuille de protection (8).

2. Procédé selon la revendication 1, **caractérisé par** l'étape supplémentaire suivante :
e) remplissage de l'évidement intérieur (3) avec un produit de remplissage (2) et contrecollage d'une feuille de recouvrement.

3. Procédé selon la revendication 1 ou 2, **caractérisé par** l'étape supplémentaire suivante :
f) découpage du contour de la forme extérieure du pansement en séparant les découpes et en ôtant les résidus de découpage en forme de grille.

4. Procédé selon la revendication 1, **caractérisé en ce que** les étapes sont effectuées dans l'ordre a) à d).

5. Procédé selon la revendication 1, **caractérisé en ce que** les étapes sont effectuées dans l'ordre b), a), c) et d).

6. Procédé selon la revendication 1, **caractérisé en ce que** les étapes a) et b) sont effectuées simultanément.

7. Procédé selon la revendication 1, **caractérisé en ce que** les étapes sont effectuées dans l'ordre a), c), d) et b).

8. Procédé selon la revendication 1, dans lequel la bande continue (1) comprend une couche support (4), une couche adhésive (5) et une matrice (6), **caractérisé en ce que** l'évidement intérieur (3) de la couche adhésive (5) est découpé à travers la matrice (6) et ensemble avec celle-ci.

9. Procédé selon l'une des revendications 1 à 8, **caractérisé en ce que** l'évidement intérieur (3) est rempli d'un produit de remplissage (2) qui contient une préparation fluide ou pulvérulente avec un principe actif pharmaceutique capable d'une action locale, régionale, topique et/ou systémique.

10. Procédé selon la revendication 9, **caractérisé en ce que** le principe actif pharmaceutique est choisi dans le groupe comprenant l'acide salicylique, l'acide lactique, la 5-fluoruracil, la Capsaicine, l'acide acétylsalicylique ou le vanillylamide de l'acide nonylique.
